# FASCICULE DE BREVET EUROPEEN

(11) **EP 2 313 389 B1**
(45) Date de publication et mention de la délivrance du brevet: **27.06.2012**
(21) Numéro de dépôt: 09806436.3
(22) Date de dépôt: 13.08.2009
(51) Int. Cl.: C07D 319/12

(54) **PROCEDE D'OBTENTION DE LACTIDE**
VERFAHREN ZUR GEWINNUNG VON LACTID
METHOD OF OBTAINING LACTIDE

(30) Priorité: 14.08.2008 BE 200800450
(43) Date de publication de la demande: 27.04.2011
(73) Titulaire: Futerro S.A., 7760 Escanaffles (BE)
(72) Inventeur: MARIAGE, Pierre-Antoine, B-7760 Escanaffles (BE); HOTTOIS, Delphine, B-7760 Escanaffles (BE); COSZACH, Philippe, B-7760 Escanaffles (BE)
(74) Mandataire: Leyder, Francis
(86) Numéro de dépôt international: PCT/EP2009/060501
(87) Numéro de publication internationale: WO 2010/018209

(56) Documents cités:
- US-A- 5 214 159
- US-A- 5 319 107
- US-A- 5 463 086

## Description

La présente invention concerne un procédé d'obtention de lactide à l'aide d'un solvant de la famille des éthers à partir d'un mélange se présentant sous forme liquide comprenant du lactide, du méso-lactide et d'autres impuretés.

Le développement des bioplastiques est actuellement en plein essor. L'utilisation de bioplastiques tel que par exemple le polylactide (PLA) dans le domaine des emballages se développe activement.

Le PLA est un polyester aliphatique à base d'acide lactique, ce dernier étant obtenu par fermentation de sucres et/ou d'amidon. Le polylactide est donc issu de ressources végétales renouvelables et est biodégradable par compostage.

La production du PLA peut se faire au départ du dimère cyclique de l'acide lactique appelé lactide.

Les deux formes optiquement actives d'acide lactique (L-LA) et (D-LA) peuvent donner un lactide (LD ou dimère cyclique) sous 3 formes stéréoisomériques : avec 2 molécules d'acide D-lactique (D,D-lactide ou D-LD), avec 2 molécules d'acide L-lactique (L,L-lactide ou L-LD) ou avec une molécule de chaque (méso-lactide ou méso-LD). On rencontre aussi le mélange racémique ((D,L)-lactide) caractérisé par une température de fusion (Tf = 126°C) supérieure à celle du L-LD ou D-LD (Tf = 97°C) et supérieure à celle du méso-LD (Tf=50°C).

Actuellement, les 2 grandes méthodes de production de lactide se distinguent essentiellement par le degré de polymérisation moyen (DP) des oligomères de l'étape de condensation.

La première consiste à extraire l'eau d'une solution d'acide lactique jusqu'à obtenir des oligomères avec 8 ≤ DP ≤ 25. Ensuite, on dépolymérise ces oligomères (réaction de back-bitting) avec un catalyseur acide de Lewis, soit sous une pression réduite à température plus ou moins élevée, soit sous un flux d'azote. Ce procédé se réalise dans des conditions drastiques qui influent sur la pureté optique du lactide (pourcentage élevé de racémisation).

La seconde méthode utilise un oligomère avec 1,5 ≤ DP ≤ 2,5 produit en phase vapeur à température élevée ou en phase liquide en présence d'un co-solvant formant un azéotrope avec l'eau. Les principaux inconvénients sont la présence d'un solvant souvent aromatique et de haut point d'ébullition, une température de réaction supérieure à 180°C, un manque de sélectivité et une quantité non négligeable d'impuretés.

De manière générale, le lactide obtenu par diverses voies de synthèse doit être purifié afin d'obtenir une pureté suffisante avant de procéder à sa polymérisation par ouverture de cycle.

Ces étapes de synthèse et de purification entraînent donc la formation d'une quantité économiquement importante de sous-produits contenant du L-lactide, du D-lactide, du méso-lactide, du mélange racémique ((D,L)-lactide), de l'acide lactique, d'autres oligomères de l'acide lactique, des produits de dégradation thermique de l'acide lactique et d'autres impuretés plus spécifiques aux procédés de synthèse du lactide telle que par exemple l'acide 2 éthylhexanoïque provenant de la dégradation de l'octanoate d'étain pouvant être utilisé comme catalyseur pour la synthèse du lactide.

Ces sous-produits peuvent être :
- hydrolysés de manière à récupérer l'acide lactique présent, la solution pouvant être recyclée en amont de la synthèse du lactide. Ceci nécessite la reconduite du processus complet, ce qui n'est pas avantageux d'un point de vue du rendement énergétique et donc pas économique.
- extraits avec de l'eau. Le lactide n'étant pas soluble, il précipite et peut être récupéré après filtration et séchage. Ceci est divulgué dans le document EP 1 276 735. Cependant, le lactide forme avec l'eau un complexe qui se dégrade progressivement en un dimère de l'acide lactique. Ceci entraîne d'une part une perte importante de rendement et d'autre part, engendre une pureté insuffisante du lactide après séchage (contamination par de l'acide lactoyl-lactique). De plus, les oligomères d'acide lactique de taille supérieure à 2 unités sont également peu solubles dans l'eau et bien que liquides, leurs filtrabilités restent faibles dû à leurs viscosités;
- extraits avec des solvants organiques plus ou moins apolaires tels que le toluène, l'ethyl-acétate ou des mélanges de ceux-ci. Cependant, lors d'une extraction, la quantité d'impuretés solubilisées est fonction de la polarité et du ratio solvant/sous-produit. Lorsque la part d'impureté dans le sous-produit augmente, il est nécessaire d'utiliser un solvant à polarité supérieure et/ou une fraction plus importante du solvant pour éviter de dépasser le seuil de saturation de la phase d'extraction. En augmentant la polarité et/ou la quantité de la phase d'extraction, la quantité de lactide qui se solubilise augmente et la perte est plus importante. En conséquence, plus le sous-produit est pauvre en lactide (et donc riche en impuretés), plus la perte de lactide par ce type de solvant augmente et plus faible est le rendement.

La présente invention pallie ces inconvénients tout en permettant d'obtenir le lactide au départ de sous-produits avec un rendement acceptable industriellement.

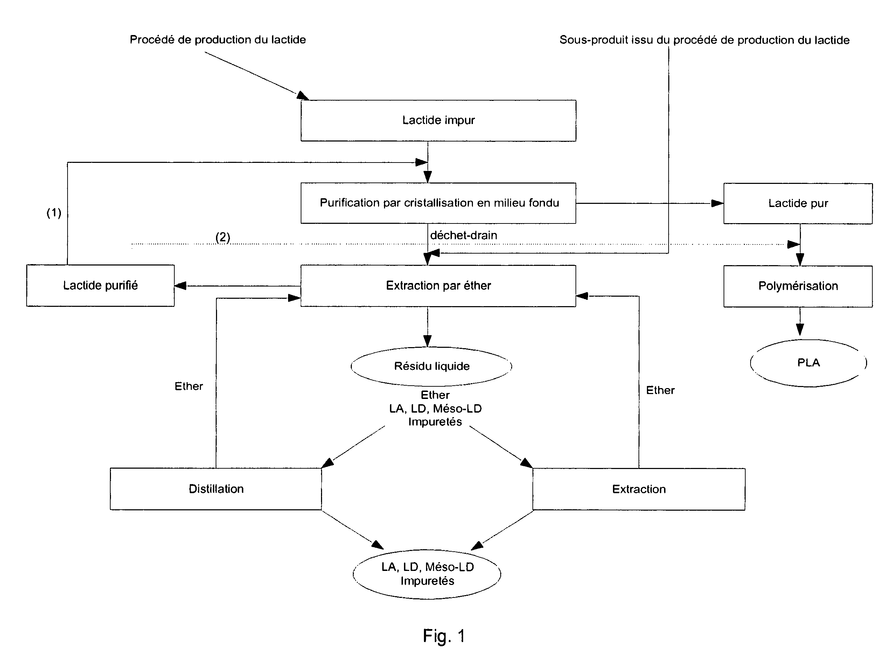

La figure 1 décrit de manière schématique un mode d'exécution de l'invention.

On entend par lactide, le diester cyclique de l'acide lactique représenté par la formule générale suivante : 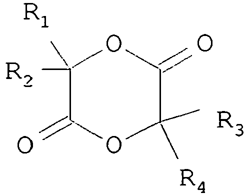 dans laquelle R1=R3=H et R2=R4=CH3.

Dans la présente invention, on entend par lactide, l'une des deux formes stéréoisomériques (le L-LD ou le D-LD) et non le méso-LD.

Le but de la présente invention est de fournir un procédé d'obtention de lactide, plus particulièrement de L-lactide ou de D-lactide, à partir d'un mélange se présentant sous forme liquide comprenant du lactide, du méso-lactide, et d'autres impuretés à l'aide d'un solvant de la famille des éthers.

Le document US 5,214,159 divulgue un procédé pour la production de mésolactide au départ d'un mélange de méso-lactide et de D,L-lactide. Le mélange est précipité sous forme cristalline dans un alcool. Les cristaux de mésolactide et de D,L-lactide obtenus sont ensuite recristallisés dans l' alcool puis dissous dans un éther aliphatique. Le D,L-lactide cristallise dans l'éther. La liqueur mère restante, consistant en un mélange de méso-lactide et de D,L-lactide dans un rapport d'environ 60/40, est concentrée par évaporation et le résidu est soumis à la distillation. Ce document ne divulgue pas la précipitation du lactide dans l'éther au départ d'un lactide impur présent sous forme liquide.

Carothers et al. divulguent dans le Journal of American Chemistry Society, volume 54, 1932, pages 761-772 la purification du lactide par cristallisation dans l'éther. Ce document ne divulgue pas la précipitation du lactide dans l'éther au départ d'un lactide impur présent sous forme liquide.

EP 0 588 222 divulgue un procédé pour séparer le lactide du méso-lactide par lavage à l'éther d'un lactide impur présent sous forme solide. Ce document ne divulgue pas la précipitation du lactide dans l'éther au départ d'un lactide impur présent sous forme liquide.

US 5,463,086 divulgue dans l'exemple comparatif 4 un procédé pour séparer le L-lactide du méso-lactide par dissolution d'un brut de lactide (2,19 gr) dans le diéthyl éther (41,43 gr) suivi d'une cristallisation. Ce document ne divulgue pas de précipitation de lactide dans l'éther au départ d'un lactide impur sous forme liquide et d'éther présents dans les proportions de la présente invention.

US 5,319,107 divulgue dans les exemples 1 et 2 la dissolution d'un résidu de lactide, d'acide lactique et d'oligomères dans le diethyl ether suivi d'une cristallisation du lactide par incubation de la solution à 4°C. Un lactide d'une pureté supérieure à 90%, voire supérieure à 95% avec un rendement de 13% a été obtenu. Ce document ne divulgue pas la précipitation du lactide au départ d'un lactide impur présent sous forme liquide.

La présente invention fournit un procédé d'obtention de lactide au départ d'un mélange se présentant sous forme liquide comprenant du lactide, du mésolactide et d'autres impuretés, le procédé comprenant les étapes suivantes:
(a) addition au mélange d'un éther caractérisé en ce que ledit mélange et l'éther se présentent tous deux sous la forme liquide à la température de mélange et en ce que l'éther est ajouté au mélange dans un rapport massique éther/mélange allant de 0,5:1 à 10:1,
(b) refroidissement optionnel du mélange et de l'éther à l'issue de l'étape (a),
(c) précipitation d'un lactide purifié dans une phase liquide,
(d) séparation du mélange, issu de l'étape (c), et obtention d'un gâteau humide riche en lactide et d'une phase liquide.

Le procédé de l'invention permet l'obtention aussi bien du D-lactide que du L-lactide.

Le mélange utilisé dans la présente invention peut être issu de toute synthèse du lactide, connue de l'homme de l'art, au départ de l'acide lactique et/ou de ses sels et/ou de ses esters ou être issu de résidus de procédés de purification du lactide comme par exemple la distillation ou la cristallisation en milieu fondu.

Dans la présente invention, on entend par "autres impuretés": l'acide lactique, les autres oligomères de l'acide lactique, les produits de dégradation thermique de l'acide lactique, les esters de l'acide lactique et leurs oligomères respectifs, les produits de dégradation thermique des esters de l'acide lactique, les sels de l'acide lactique et leurs oligomères respectifs, les produits de dégradation thermique des sels de l'acide lactique, l'eau, l'alcool et des résidus de catalyseur tel que par exemple l'acide 2-ethylhexanoïque.

Le mélange de départ typique comprend entre 30 et 80% de L-lactide, de préférence entre 40 et 70%, entre 0 et 2% d'eau, de préférence entre 0 et 1 %, entre 5 et 50% d'acide lactique et autres oligomères de l'acide lactique (LₙA avec n inférieur ou égal à 5), entre 0 et 30 % de méso-lactide et entre 0 et 30 % de résidus de catalyseur tel que l'acide 2-ethylhexanoïque et/ou des produits de dégradation thermique de l'acide lactique.

Ce mélange peut être issu de la purification par cristallisation à l'état fondu d'un lactide impur (aussi appelé brut de lactide). La cristallisation à l'état fondu du lactide impur conduit d'une part à la formation d'un lactide purifié sous forme cristalline ayant une teneur riche en lactide, généralement supérieure à 99%, de préférence supérieure à 99,5% et une teneur pauvre en mésolactide, généralement inférieure à 1 %, de préférence inférieure à 0,5% et d'autre part à la formation d'une fraction résiduelle liquide appelée aussi résidu ou drain comprenant un mélange de lactide, du mésolactide et d'autres impuretés dans les proportions telles que décrites dans le paragraphe précédent.

Dans la présente invention, l'éther et le mélange comprenant le lactide, le mésolactide et les autres impuretés sont tous deux sous forme liquide à la température de mélange comprise entre 50°C et 90°C, de préférence comprise entre 55°C et 80°C. Dès que l'éther est ajouté au mélange, le lactide précipite instantanément dans l'éther.

Selon un mode de réalisation de l'invention, lorsque le mélange de départ contient une quantité en méso-lactide non négligeable, par exemple une quantité supérieure à 10%, l'éther est ajouté au mélange à une température supérieure à la température de précipitation du méso-lactide dans l'éther, afin d'éviter la précipitation du méso-lactide, et inférieure à la température d'ébullition de l'éther.

Après addition de l'éther, le mélange peut être agité et est maintenu à température, de préférence au-dessus de la température de précipitation du mésolactide dans l'éther pour éviter la précipitation de celui-ci, jusqu'à précipitation complète du lactide. La phase liquide comprend le méso-lactide et les autres impuretés.

En fonction de l'éther utilisé, il peut s'avérer nécessaire de refroidir entre 0°C et la température ambiante le mélange auquel de l'éther a été ajouté pour précipiter le lactide. Le mélange, constitué d'un lactide précipité dans une phase liquide, est ensuite séparé à une température suffisamment basse, de préférence entre 15°C et 30°C, pour permettre une récupération quantitative du lactide mais pas trop faible afin de permettre une élimination aisée des autres impuretés se présentant sous forme non solide.

Lorsque le lactide a précipité dans la phase liquide, le mélange est séparé. De préférence, la séparation se fait à chaud, c'est à dire à une température supérieure à la température de précipitation du méso-lactide dans l'éther, afin d'éliminer au maximum le méso-lactide dans la phase liquide. La séparation peut se faire par toute technique connue de l'homme de l'art pour les séparations solide/liquide telle que par exemple la filtration, la centrifugation, l'essorage. Un gâteau humide riche en lactide et une phase liquide sont ainsi obtenus.

Le gâteau humide obtenu après séparation est ensuite préférentiellement séché à une température produit inférieure à la température de fusion du lactide. Si le gâteau humide contient du méso-lactide résiduel, la température de séchage est de préférence inférieure à la température de fusion du mésolactide. Le séchage peut se faire par exemple sous vide.

Le procédé de l'invention permet l'obtention d'un lactide purifié ayant une teneur en lactide comprise entre 80 et 99,5% en poids.

Le degré de pureté du lactide peut être amélioré par plusieurs traitements successifs à l'éther du gâteau riche en lactide. Le gâteau riche en lactide peut être chauffé jusqu'à ce qu'il devienne liquide et traité avec une nouvelle addition d'éther ou lavé tel quel, c'est à dire sans passer à l'état fondu, avec une nouvelle addition d'éther.

De préférence, l'éther utilisé dans le procédé de l'invention est un éther de formule générale

R¹-O-R²

dans laquelle R¹ et R² représentent, indépendamment l'un de l'autre, un groupe alkyl ou alkenyl linéaire ou branché ayant de 1 à 4 atomes de carbone, un groupe alcanol ayant de 1 à 4 atomes de carbone,
un groupe aryl,
un groupe arylalkyl,
un groupe cycloalkyl ayant de 4 à 6 atomes de carbone
ou dans laquelle R¹ et R² forment une structure cyclique ayant de 2 à 6 atomes de carbone

De préférence, les éthers ayant au total 6 atomes de carbone dans la formule générale décrite ci-dessus sont utilisés de manière à faciliter l'extraction de ceux-ci.

Des éthers tels que le dipropyl éther, le diisopropyl éther, le dibutyl éther, le dipropyl éther, le diéthyl éther, le méthyl ter-butyl éther, l'éthyl propyl éther, l'éthyl ter-butyl éther, le tétrahydrofurane, le furane, l'éther monométhylique de l'éthylène glycol, l'éther monobutylique de l'éthylène glycol, l'éther éthylvinylique, l'éther dibenzylique, le méthyl amyl éther peuvent être utilisés dans le procédé de l'invention. De préférence, le dipropyl éther, le diisopropyl éther, le diéthyl éther et le tétrahydrofurane sont utilisés.

L'éther est ajouté au mélange dans un rapport massique éther/mélange allant de 0.5:1 à 10:1, de préférence dans un rapport massique éther/mélange allant de 1:1 à 5:1. Le procédé de l'invention permet l'obtention d'un lactide purifié au départ d'un lactide impur sous forme liquide par traitement de ce dernier avec une quantité limitée d'éther, rendant ainsi le procédé particulièrement applicable industriellement contrairement aux procédés connus faisant appel à une dissolution du lactide impur dans l'éther. Ces procédés requièrent l'utilisation de quantités importantes d'éther, ce qui les rend difficilement exploitable d'un point de vue industriel. L'utilisation de quantités importantes d'éther nécessaire pour la dissolution d'un lactide impur est exemplifiée ultérieurement dans la demande.

La phase liquide, obtenue à l'issue de l'étape (d), composée d'éther, de mésolactide et d'autres impuretés telles que définies précédemment peut être, selon un mode de réalisation, envoyée dans une colonne d'extraction ou dans une succession de mixer-settler où elle est mise en contact avec une phase aqueuse ou autre en vue de récupérer le méso-lactide et les autres impuretés (étape e).

La phase éther après ou sans passage par une étape de régénération/nettoyage du solvant (procédé acide citrique Bateman) peut être récupérée pour réaliser une nouvelle extraction du mélange initial (étape f).

La phase aqueuse comprenant le lactide résiduel, le méso-lactide ainsi que les autres impuretés peut être recyclée, si nécessaire après purification (résines, charbon, filtration, ...), en amont de la synthèse de lactide (étape g).

Selon un autre mode de réalisation de l'invention, la phase liquide composée d'éther, de méso-lactide et d'autres impuretés obtenue à l'issue de l'étape (d) du procédé de l'invention peut être envoyée dans une colonne de distillation avec garnissage dans laquelle l'éther est distillé en tête de colonne tandis que la phase chargée de méso-lactide et d'autres impuretés sort en bas de colonne (étape e'). Cette dernière peut être hydrolysée avec de l'eau pour régénérer par exemple de l'acide lactique (étape f) qui pourra être utilisé ultérieurement, après purification. L'éther récupéré peut ensuite être utilisé pour réaliser une nouvelle extraction (étape g').

Quel que soit le mode de réalisation décrit ci-dessus, une amélioration du procédé consiste, préalablement à l'étape (e) et (e'), à refroidir la phase liquide composée de l'éther chargée d'impuretés issue de l'étape (d) en dessous de 30°C jusqu'à précipitation du lactide et du méso-lactide résiduels (étape i) et à filtrer à nouveau de manière à récolter un nouveau gâteau (étape ii).

Le lactide issu du procédé de l'invention peut être ultérieurement purifié par un procédé de cristallisation en milieu fondu afin d'obtenir un lactide d'une pureté suffisante pour, par exemple, la synthèse du PLA par ouverture de cycle. Dans ce cas, le gâteau humide riche en lactide obtenu à l'issue de l'étape (d) du procédé de l'invention ou le gâteau sec obtenu après séchage du gâteau humide peut être fondu et recyclé comme fraction principale ou fraction intermédiaire d'un procédé de purification par cristallisation en milieu fondu. Cette option permet d'envisager d'un point de vue économique une exploitation industrielle d'un procédé de purification du lactide par cristallisation en milieu fondu sans utilisation d'une pré-purification par distillation du lactide impur obtenu à l'issue de sa synthèse.

Une pureté suffisante implique une teneur entre 99,0 et 99,9% en lactide, préférablement entre 99,5 et 99,9%, une teneur entre 0 et 0,5% en méso-lactide, préférablement entre 0 et 0,2%, une teneur entre 0 et 100 ppm en eau, préférablement entre 0 et 50 ppm.

Dans la cristallisation en milieu fondu, le lactide qui peut être issu du procédé de l'invention, est fondu et subit un refroidissement contrôlé qui initie sa cristallisation (un ensemencement de cristaux est parfois nécessaire) sur une paroi et/ou directement dans le fondu. Les impuretés sont ainsi concentrées dans la phase liquide.

Après la phase de cristallisation, la phase liquide est éliminée soit par gravité soit par toute technique connue de l'homme de l'art pour les séparations solide/liquide comme par exemple filtration, filtration sous vide, filtration sous pression ou centrifugation.

De sorte à pouvoir éliminer le film d'impuretés enrobé à la surface des cristaux, des technologies plus pointues sont requises comme la refonte partielle des cristaux dont le liquide obtenu est évacué par gravité ou l'utilisation d'une colonne de lavage, avec transport forcé ou pas telle que, par exemple, celles mentionnées dans "Melt Crystallization-Fundamentals Equipment and Applications" edité par Jouchim Ulrich, Herke Glade, Shaker Verlag, 2003 et dans "Melt Crystallization Technology", G.F. Arkenbout, Technomic Publishing Company Inc., 1995.

Les cristaux ainsi purifiés (l'opération peut être répétée jusqu'à atteindre la pureté requise) peuvent être ensuite refondus et valorisés comme intermédiaire réactionnel pour la synthèse du PLA par ouverture de cycle. La phase liquide éliminée comporte un concentrat d'impuretés mais également une quantité non négligeable de lactide et de méso-lactide. Le traitement de cette phase liquide suivant le procédé de l'invention permet d'extraire une partie importante du lactide et du méso-lactide présents.

### Exemples

Dans tous les exemples ci-dessous, le résidu ou drain provient de la purification par cristallisation en milieu fondu d'un lactide impur et les teneurs des divers constituants ont été déterminés par chromatographie en phase gazeuse après sylilation des composés carboxylés.

### Exemple 1

Le résidu ou drain a été traité avec l'éther diisopropylique. A cet effet, 100 g de drain ont été préalablement chauffés à 85°C de manière a obtenir un drain se présentant sous la forme liquide. 100 gr d'éther diisopropylique ont ensuite été ajoutés et mélangés au drain. Le mélange a été porté à 55°C durant 1 h sous agitation puis a été filtré sur Buchner à 55°C pour éviter que le mésolactide ne précipite. Le drain précipité a été récupéré comme gâteau de filtration et l'éther diisopropylique comme filtrat.

Le drain précipité a été séché sous vide à température ambiante et les teneurs des divers constituants ont été déterminées. Les résultats sont repris dans le tableau 1.

L'extraction à l'éther diisopropylique permet d'augmenter la teneur en L-lactide de 61,9 à 88,9%. Le rendement de récupération du L-lactide est de 64,2%. La majorité du méso-lactide, de l'acide 2-éthyl hexanoique, et des oligomères de l'acide lactique se retrouvent dans le filtrat. Le complexe lactide+eau se décompose en dimère d'acide lactique et passe également dans la phase de l'ether diisopropylique. Cette extraction à l'éther diisopropylique permet une très bonne séparation du lactide des autres éléments.

| **TABLEAU** 1 | Drain initial | Lactide purifié après séchage | Filtrat |
|---|---|---|---|
| Constituants | % massique | % massique | % massique |
| Acide lactique | 1,7 | 0,6 | 3,6 |
| Méso-lactide | 12,3 | 2,8 | 22,2 |
| Dimère d'acide lactique | 2,6 | 2,2 | 9,8 |
| L-lactide | 61,9 | 88,9 | 29,4 |
| Acide 2-éthylhexanoique | 11,8 | 3,3 | 24,6 |
| Lactide + eau | 5,4 | 0,2 | 0,1 |
| Trimère d'acide lactique | 1,7 | 0,8 | 5,5 |
| Tétramère d'acide lactique | 1,4 | 0,6 | 2,6 |
| Pentamère d'acide lactique | 1,2 | 0,6 | 2,2 |
| Enrichissement % | + 27 % | | |
| Rendement L-lactide % (L-LD out /L-LD in) | + 64,2 % | | |

La totalité du filtrat (125,5 gr) chargé d'acide lactique a été ensuite envoyé en continu dans une colonne de distillation avec un garnissage. La température de la colonne était de 118°C dans le bas et de 72°C dans le haut. L'éther diisopropylique a été récupéré en tête de colonne tandis que la phase contenant le méso-lactide et les autres impuretés a été récoltée dans le bas de la colonne. Des 125,5 gr de filtrat, 43 gr ont été récoltés. Cette phase contenant l'acide lactique a ensuite été hydrolysée avec de l'eau à 80°C pendant 2 heures pour être recyclée en amont de la synthèse de lactide.

L'éther diisopropylique peut ensuite être récupéré pour réaliser une nouvelle extraction du drain.

### Exemple 2

Dans cet exemple, le lactide purifié et séché obtenu dans l'exemple 1 a été lavé avec de l'éther diisopropylique. Pour ce faire, le lactide purifié a été mélangé à température ambiante avec de l'éther diisopropylique dans des quantités équivalentes pendant 1 heure. Le mélange a été filtré à température ambiante. Le lactide qui a précipité dans le mélange a été récupéré comme gâteau et séché selon des conditions identiques à celles de l'exemple 1

Les teneurs des divers constituants ont été déterminées. Les résultas sont repris dans le tableau 2.

| **TABLEAU 2** | Lactide purifié | Lactide purifié après lavage | Filtrat |
|---|---|---|---|
| Constituants | % massique | % massique | % massique |
| Acide lactique | 0,6 | 0,1 | 2,1 |
| Méso-lactide | 2,8 | 2,8 | 4,6 |
| Dimère d'acide lactique | 2,2 | 0,5 | 12,8 |
| L-lactide | 88,9 | 95,6 | 61,5 |
| Acide 2-éthylhexanoique | 3,3 | 0,3 | 10,7 |
| Lactide + eau | 0,2 | 0,1 | 0 |
| Trimère d'acide lactique | 0,8 | 0,1 | 4,5 |
| Tétramère d'acide lactique | 0,6 | 0,3 | 2 |
| Pentamère d'acide lactique | 0,6 | 0,2 | 1,8 |
| Enrichissement % | + 6,7 % | | |
| Rendement L-lactide % (L-LD out /L-LD in) | + 67,92 % | | |

### Exemples 3 à 6

Dans ces exemples, le résidu ou drain a été traité avec l'éther diisopropylique à différentes concentrations.

A cet effet, le drain a été préalablement chauffé à 85°C de manière à obtenir un drain sous forme liquide. 175 gr de drain liquide ont été respectivement mélangés à 87,5 gr, 175 gr, 350 gr et 875 gr d'éther diisopropylique à température ambiante. Dès le mélange, une précipitation sélective du lactide présent dans le drain a été observée. Le mélange a été progressivement refroidi jusqu'à 4°C durant 1h sous agitation puis a été filtré à température ambiante sur Buchner. Le drain précipité a été récupéré comme gâteau de filtration et l'éther diisopropylique chargé en impuretés comme filtrat.

Le drain précipité a ensuite été séché sous vide à température ambiante et les teneurs des divers constituants ont été déterminées. Les résultats sont repris dans le tableau 3.

| **TABLEAU 3** | Drain | Example 3 IPE/drain 0.5/1 | Exemple 4 IPE/drain 1/1 | Exemple 5 IPE/drain 2/1 | Exemple 6 IPE/drain 5/1 |
|---|---|---|---|---|---|
| constituants | % massique | % massique | % massique | % massique | % massique |
| Acide lactique | 2,1 | 0,9 | 0,3 | 0,3 | 0,4 |
| Méso-lactide | 12,8 | 11,4 | 12,7 | 10,9 | 4,9 |
| Dimère acide lactique | 3,9 | 0,2 | 0,2 | 0,2 | 0,2 |
| L-lactide | 60,7 | 83,1 | 86,5 | 88,2 | 93,2 |
| Acide 2-éthylhexanoique | 12,7 | 1,8 | 0,3 | 0,4 | 0,6 |
| Lactide + eau | 4,4 | 0,4 | 0 | 0 | 0,7 |
| Trimère acide lactique | 1,5 | 0,9 | 0 | 0 | 0 |
| Tétramère acide lactique | 1,1 | 0,7 | 0 | 0 | 0 |
| Pentamère acide lactique | 0,8 | 0,6 | 0 | 0 | 0 |
| Enrichissement % | | 22,4 | 25,8 | 27,5 | 32,5 |
| Rendement L-LD % | | 67,21 | 72,55 | 79,26 | 84,36 |

### Exemple 7

Dans cet exemple, 200 gr de drain ont été préalablement fondu à 85°C puis mélangés avec 200 gr d'éther diisopropylique à 60°C. Après précipitation du lactide, le mélange est filtré à chaud sur Buchner afin de récupérer l'éther comme filtrat (filtrat initial). Ce filtrat dont la composition est mentionnée dans le tableau 4 a été refroidi à température ambiante pendant 1 heure puis à son tour filtré à température ambiante sur Buchner (filtrat après traitement). Les teneurs des divers constituants de ce filtrat ont été déterminées. Le gâteau obtenu après cette dernière filtration a été séché sous vide à température ambiante (lactide purifié après séchage) et les teneurs des divers constituants ont été déterminées (tableau 4).

| **TABLEAU 4** | Filtrat initial | Lactide purifié après séchage | Filtrat après traitement |
|---|---|---|---|
| Constituants | % massique | % massique | % massique |
| Acide lactique | 5,3 | 0,2 | 7,9 |
| Méso-lactide | 14 | 6,2 | 15,9 |
| Dimère d'acide lactique | 15 | 0,3 | 29,1 |
| L-lactide | 29,4 | 91,8 | 0,5 |
| Acide 2-éthylhexanoique | 19,6 | 0,6 | 28,7 |
| Lactide + eau | 0 | 0,1 | 0 |
| Trimère d'acide lactique | 10,6 | 0,2 | 11,3 |
| Tétramère d'acide lactique | 3,4 | 0,3 | 3,6 |
| Pentamère d'acide lactique | 2,7 | 0,3 | 3 |
| Enrichissement % | + 62,4 % | | |
| Rendement L-lactide % (L-LD out /L-LD in) | +11,05% | | |

### Exemple 8

Un drain a été fondu à 85°C puis traité respectivement avec une quantité équivalente d'éther diisopropylique (IPE), de tétrahydrofurane (THF) et de 1,2-diméthoxyéthane.

L'extraction à l'IPE a été réalisée dans les mêmes conditions que celles mentionnées dans l'exemple 1.

Pour l'extraction au THF, après avoir mélangé le drain avec une quantité équivalente d'éther, le mélange a été placé à -20°C pendant une heure pour initier la précipitation du lactide et ensuite filtré. Le gâteau de filtration a ensuite été séché sous vide à température ambiante.

Le 1,2-dimethoxyethane ne permet pas l'extraction du lactide au départ du drain initial faute de cristallisation du L-lactide aussi bien à température ambiante qu'à plus basse température.

Les résultats des extractions à l'IPE et au THF sont repris dans le tableau 5.

| **TABLEAU 5** | Drain initial | Lactide purifié après séchage (IPE) | Lactide purifié après séchage (THF) |
|---|---|---|---|
| Constituants | % massique | % massique | % massique |
| Acide lactique | 4,5 | 0,5 | 0,1 |
| Méso-lactide | 9,4 | 2,1 | 1,3 |
| Dimère d'acide lactique | 8,6 | 5,5 | 0,2 |
| L-lactide | 52,0 | 89,2 | 98,1 |
| Acide 2-éthylhexanoique | 15,8 | 1,4 | 0,2 |
| Lactide + eau | 3,8 | 0,2 | 0,0 |
| Trimère d'acide lactique | 4,6 | 0,7 | 0,1 |
| Tétramère d'acide lactique | 1,1 | 0,3 | 0,0 |
| Pentamère d'acide lactique | 0,2 | 0,1 | 0,0 |
| Pentamère d'acide lactique | 0,2 | 0,1 | 0,0 |

### Exemples 9-10

Un drain a été traité avec l'éther diisopropylique selon 2 procédures différentes: le lavage (exemple 9 comparatif) et l'extraction (exemple 10 selon l'invention).

Pour l'extraction, 100 gr de drain ont été fondu à 85°C puis mélangés avec 100 gr d'éther diisopropylique à température ambiante. Le mélange a été progressivement refroidi jusqu'à 4°C durant 1 heure sous agitation puis filtré à température ambiante sous Buchner. Le drain précipité a été séché sous vide à température ambiante et les teneurs des divers constituants déterminées. Les résultats sont repris dans le tableau 6.

Pour le lavage, 100 gr d'éther diisopropylique ont été ajoutés à 100 gr de drain solide à température ambiante. Le mélange a été agité durant 1 heure à température ambiante puis filtré. Le drain précipité a été récupéré comme gâteau de filtration. Le gâteau de filtration a été séché sous vide à température ambiante. Les résultats sont repris dans le tableau 6.

| | | Example 9 | Example 10 |
|---|---|---|---|
| **TABLEAU 6** | Lactide impur | Lactide purifié après lavage (comparatif) | Lactide purifié après extraction (invention) |
| constituants | % massique | % massique | % massique |
| Acide lactique | 2,5 | 0,5 | 0,1 |
| Méso-lactide | 19,4 | 10,4 | 12,6 |
| Dimère acide lactique | 2,1 | 1,4 | 0,2 |
| L-lactide | 67,7 | 85,5 | 87 |
| Acide 2-éthylhexanoique | 5,3 | 0,7 | 0 |
| Lactide + eau | 2,7 | 0,2 | 0,1 |
| Trimère acide lactique | 0,3 | 0,7 | 0 |
| Tétramère acide lactique | 0 | 0,5 | 0 |
| Pentamère acide lactique | 0 | 0,1 | 0 |
| Rendement L-LD % | | 71,36 | 85,46 |

Le rendement de récupération en lactide est plus important lors du traitement par extraction que par lavage. Le lavage à l'éther du drain n'est pas suffisant pour la purification du lactide. Ce dernier contient encore des impuretés carboxyliques (oligomères d'acide lactique) empêchant la polymérisation du lactide. De plus, il est d'aspect jaunâtre contrairement au lactide après extraction qui est blanc.

### Exemple 11

Dans cet exemple, un drain sous forme solide a été mélangé à température ambiante avec différentes quantités d'éther diisopropylique. Pour ce faire, 100gr de drain ont été mélangés respectivement à 100gr, 200gr, 500gr, 1000gr, 2000gr, 3000 gr et 4000gr d'éther diisopropylique. Le mélange est ensuite porté à 60°C.

Aucune dissolution du drain dans l'éther diisopropylique n'a été observée excepté dans les essais où le drain était en présence de trente et quarante fois plus de solvant. Après un temps de chauffe de 6h pour les deux essais, une dissolution partielle du drain a été observée dans le premier cas et une dissolution complète du drain a été observée dans le second cas.

Pour les autres cas, on a constaté clairement la précipitation sélective du lactide qui a ensuite été filtré sur Buchner et séché sous vide à température ambiante.

## Revendications

1. Procédé d'obtention de lactide au départ d'un mélange comprenant du lactide, du méso-lactide, et d'autres impuretés, le procédé comprenant les étapes suivantes:
(a) addition au mélange d'un éther **caractérisé en ce que** ledit mélange et l'éther se présentent tous deux sous forme liquide à la température de mélange et **en ce que** l'éther est ajouté au mélange dans un rapport massique éther/mélange allant de 0,5:1 à 10:1,
(b) refroidissement optionel du mélange et de l'éther obtenu à l'issue de l'étape (a),
(c) précipitation d'un lactide purifié dans une phase liquide,
(d) séparation du mélange issu de l'étape (c) et obtention d'un gâteau humide riche en lactide et d'une phase liquide.

2. Procédé selon la revendication 1 **caractérisé en ce que** le rapport massique éther/mélange varie entre 1:1 et 5:1.

3. Procédé selon la revendication 1 ou 2 **caractérisé en ce que** le gâteau humide issu de l'étape (d) est séché à une température produit inférieure à la température de fusion du lactide de manière à obtenir un gâteau sec riche en lactide.

4. Procédé selon les revendications 1 à 3 **caractérisé en ce que** le séchage du gâteau humide se fait à une température inférieure à la température de fusion du méso-lactide.

5. Procédé selon les revendications 1 à 4 comprenant une étape additionelle consistant à laver le gâteau humide ou le gâteau sec riche en lactide avec de l'éther.

6. Procédé selon les revendications 1 à 5 comprenant une étape additionnelle consistant à fondre le gâteau humide ou le gâteau sec riche en lactide et à le recycler comme fraction principale ou fraction intermédiaire d'un procédé de purification par cristallisation en milieu fondu.

7. Procédé selon les revendications 1 à 6 **caractérisée en ce que** l'étape (d) se fait à une température supérieure à la température de précipitation du méso-lactide dans l'éther.

8. Procédé selon les revendications 1 à 7 **caractérisé en ce que** :
(e) la phase liquide composée de l'éther chargé de méso-lactide et d'autres impuretés, issue de l'étape d) après avoir récupéré le gâteau riche en lactide, est envoyée dans une colonne d'extraction ou une succession de mixer-settler où elle est mise en contact avec une phase aqueuse qui en récupère le méso-lactide et les autres impuretés,
(f) la phase éther après ou sans passage par une étape de régénération/nettoyage du solvant est récupérée pour réaliser une nouvelle extraction du mélange initial et
(g) la phase aqueuse comprenant le lactide résiduel, le mésolactide et les autres impuretés est recyclée, éventuellement après purification, en amont de la synthèse de lactide.

9. Procédé selon la revendication 1 à 7 **caractérisé en ce que** :
(i) la phase liquide composée de l'éther chargé d'impuretés, issue de l'étape d), est refroidie en dessous de 30°C jusqu'à précipitation du lactide et du méso-lactide résiduels, et
(ii) le lactide et le méso-lactide résiduels sont filtrés de manière à récolter un nouveau gâteau et une phase liquide,
(e) la phase liquide comprenant de l'éther issue de l'étape (ii) après avoir récupéré le gâteau riche en lactide, est envoyée dans une colonne d'extraction ou une succession de mixer-settler où elle est mise en contact avec une phase aqueuse qui en récupère le méso-lactide et les autres impuretés,
(f) la phase éther après ou sans passage par une étape de régénération/nettoyage du solvant est récupérée pour réaliser une nouvelle extraction du mélange initial et
(g) la phase aqueuse comprenant le lactide résiduel, le mésolactide et les autres impuretés est recyclée, éventuellement après purification, en amont de la synthèse de lactide.

10. Procédé selon les revendications 1 à 7 **caractérisé en ce que** :
(e') la phase liquide composée de l'éther chargé de méso-lactide et d'autres impuretés, issue de l'étape d) après avoir récupéré le gâteau riche en lactide, est envoyée dans une colonne de distillation avec garnissage dans laquelle l'éther est distillé en tête de colonne tandis que la phase chargée de méso-lactide et d'autres impuretés sort en bas de colonne,
(f') la phase chargée de méso-lactide et d'autres impuretés issue de l'étape (e') est hydrolysée avec de l'eau,
(g') l'éther issu de l'étape (e') est récupéré.

11. Procédé selon les revendications 1 à 7 **caractérisé en ce que**
(i) la phase liquide composée de l'éther chargé d'impuretés, issue de l'étape d), est refroidie en dessous de 30°C jusqu'à précipitation du lactide et du méso-lactide résiduels, et
(ii) le lactide et le méso-lactide résiduels sont filtrés de manière à récolter un nouveau gâteau et une nouvelle phase liquide,
(e') la phase liquide comprenant de l'éther issue de l'étape (ii) après avoir récupéré le gâteau riche en lactide, est envoyée dans une colonne de distillation avec garnissage dans laquelle l'éther est distillé en tête de colonne tandis que la phase chargée de mésolactide et d'autres impuretés sort en bas de colonne,
(f) la phase chargée de méso-lactide et d'autres impuretés issue de l'étape (e') est hydrolysée avec de l'eau,
(g') l'éther issu de l'étape (e') est récupéré.

12. Procédé selon l'une quelconque des revendications précédentes **caractérisé en ce que** l'éther est de formule générale
R¹-O-R²
dans laquelle R¹ et R² représentent, indépendamment l'un de l'autre, un groupe alkyl ou alkenyl linéaire ou branché ayant de 1 à 4 atomes de carbone,
un groupe alcanol ayant de 1 à 4 atomes de carbone,
un groupe aryl,
un groupe arylalkyl,
un groupe cycloalkyl ayant de 4 à 6 atomes de carbone ou dans laquelle R¹ et R² forment une structure cyclique ayant de 2 à 6 atomes de carbone.

## Claims

1. A method for obtaining a lactide starting from a mixture comprising a lactide, a meso-lactide, and other impurities, the method comprising the following steps:
(a) adding an ether to the mixture, **characterized in that** said mixture and the ether both appear in liquid form at the mixing temperature and **in that** the ether is added to the mixture in an ether/mixture mass ratio ranging from 0.5:1 to 10:1,
(b) optionally cooling the mixture and the ether obtained at the end of step (a),
(c) precipitating a purified lactide from a liquid phase,
(d) separating the mixture stemming from step (c) and obtaining a humid cake rich in lactide and a liquid phase.

2. The method according to claim 1, **characterized in that** the ether/mixture mass ratio varies between 1:1 and 5:1.

3. The method according to claim 1 or 2, **characterized in that** the humid cake stemming from step (d) is dried at a product temperature below the melting temperature of the lactide so as to obtain a dry cake rich in lactide.

4. The method according to claims 1 to 3, **characterized in that** the drying of the humid cake is accomplished at a temperature below the melting temperature of the meso-lactide.

5. The method according to claims 1 to 4, comprising an additional step consisting of washing the humid cake or the dry cake rich in lactide with ether.

6. The method according to claims 1 to 5, comprising an additional step consisting of melting the humid cake or the dry cake rich in lactide and of recycling it as a main fraction or an intermediate fraction of a purification method by crystallization, in a molten medium.

7. The method according to claims 1 to 6, **characterized in that** step (d) is performed at a temperature above the temperature of precipitation of the meso-lactide from ether.

8. The method according to claims 1 to 7, **characterized in that**:
(e) the liquid phase consisting of the ether loaded with meso-lactide and other impurities from step (d) after having recovered the lactide-rich cake is sent into an extraction column, or a mixer-settler succession where it is put into contact with an aqueous phase, which recovers the meso-lactide and the other impurities therefrom,
(f) the ether phase after or without passing through a step for regenerating/cleaning the solvents, is recovered in order to achieve new extraction of the initial mixture and
(g) the aqueous phase comprising the residual lactide, the meso-lactide and the other impurities is recycled, optionally after purification, upstream from the lactide synthesis.

9. The method according to claim 1 to 7, **characterized in that**:
(i) the liquid phase consisting of ether loaded with impurities, from step (d), is cooled below 30° C until precipitation of the residual lactide and meso-lactide, and.
(ii) the residual lactide and meso-lactide are filtered so as to harvest a new cake and a liquid phase,
(e) the liquid phase comprising the ether from step (ii), after the lactide-rich cake has been recovered, is sent into an extraction column, or a mixer-settler succession where it is put into contact with an aqueous phase, which recovers the mero-lactide and the other impurities therefrom,
(f) the ether phase after or without passing through a step for regenerating/cleaning the solvent is recovered for achieving a new extraction from the initial mixture and
(g) the aqueous phase comprising the residual lactide, the meso-lactide and the other impurities is recycled, optionally after purification, upstream from the lactide synthesis.

10. The method according to claims 1 to 7, **characterized in that**:
(e')the liquid phase, consisting of the ether loaded with meso-lactide and other impurities, from step (d), after the lactide-rich cake has been recovered, is sent into a packed distillation column in which the ether is distilled at the head of the column, while the phase loaded with meso-lactide and other impurities flows out at the bottom of the column,
(f) the phase loaded with meso-lactide and other impurities from step (e') is hydrolyzed with water,
(g')the ether from step (e') is recovered.

11. The method according to claims 1 to 7, **characterized in that**
(i) the liquid phase, consisting of the ether loaded with impurities, from step (d), is cooled to below 30° C until precipitation of the residual lactide and meso-lactide, and
(ii) the residual lactide and meso-lactide are filtered so as to harvest a new cake and a new liquid phase,
(e')the liquid phase comprising the ether from step (ii), after the lactide-rich cake has been recovered, is sent into a packed distillation column in which the ether is distilled at the head of the column while the phase loaded with meso-lactide and other impurities flows out at the bottom of the column,
(f) the phase loaded with meso-lactide and other impurities from step (e') is hydrolyzed with water,
(g')the ether from step (e') is recovered.

12. A method according to any of the preceding claims, **characterized in that** the ether is of the general formula
R¹-O-R²
wherein R¹ and R² represent independently of each other,
a linear or branched alkyl or alkenyl group, having from 1 to 4 carbon atoms,
an alkanol group having from 1 to 4 carbon atoms,
an aryl group,
an arylalkyl group,
a cycloalkyl group having from 4 to 6 carbon atoms
or wherein R¹ and R² form a cyclic structure having from 2 to 6 carbon atoms.

## Patentansprüche

1. Verfahren zur Gewinnung von Lactid aus einem Gemisch, das Lactid, meso-Lactid und sonstige Unreinheiten umfasst, wobei das Verfahren die folgenden Schritte umfasst:
(a) Hinzufügen eines Ethers zum Gemisch, **dadurch gekennzeichnet, dass** beide, das Gemisch und der Ether, bei der Gemischtemperatur eine flüssige Form aufweisen und dass der Ether dem Gemisch in einem Massenverhältnis Ether/Gemisch von 0,5:1 bis 10:1 hinzugefügt wird,
(b) optionale Abkühlung des Gemischs und des Ethers, gewonnenen von Schritt (a),
(c) Ausfällen eines gereinigten Lactids in einer flüssigen Phase,
(d) Trennung des Gemischs von Schritt (c) und Gewinnung eines lacidreichen feuchten Kuchens und einer flüssigen Phase.

2. Verfahren nach Anspruch 1, **dadurch gekennzeichnet, dass** das Massenverhältnis Ether/Gemisch zwischen 1:1 1 und 5:1 schwankt.

3. Verfahren nach Anspruch 1 oder 2, **dadurch gekennzeichnet, dass** der feuchte Kuchen von Schritt (d) bei einer Produkttemperatur unter der Fusionstemperatur des Lactids getrocknet wird, um einen lactidreichen trockenen Kuchen zu gewinnen.

4. Verfahren nach den Ansprüchen 1 bis 3, **dadurch gekennzeichnet, dass** das Trocknen des feuchten Kuchens bei einer Temperatur unter der Fusionstemperatur des meso-Lactids erfolgt.

5. Verfahren nach den Ansprüchen 1 bis 4, das einen zusätzlichen Schritt umfasst, der darin besteht, den feuchten Kuchen oder den trockenen Kuchen reich an Lactid mit Ether zu waschen.

6. Verfahren nach den Ansprüchen 1 bis 5, das einen zusätzlichen Schritt umfasst, der darin besteht, den feuchten Kuchen oder den trockenen Kuchen reich an Lactid aufzulösen und ihn als Hauptfraktion oder Zwischenfraktion eines Reinigungsverfahrens durch Schmelzkristallisation zu recyceln.

7. Verfahren nach den Ansprüchen 1 bis 6, **dadurch gekennzeichnet, dass** der Schritt (d) bei einer Temperatur über der Ausfälltemperatur des meso-Lactids im Ether erfolgt.

8. Verfahren nach den Ansprüchen 1 bis 7, **dadurch gekennzeichnet, dass**:
(e) die flüssige Phase, die aus dem mit meso-Lactid und sonstigen Unreinheiten beschickten Ether zusammengesetzt ist, von Schritt d) nach Aufbereitung des lactidreichen Kuchens in eine Extraktionssäule oder eine Mixer-Settler-Strecke geschickt wird, wo sie mit einer wässrigen Phase in Kontakt versetzt wird, die daraus das meso-Lactid und die sonstigen Unreinheiten aufbereitet,
die Ether-Phase nach oder ohne Absolvieren eines Schritts der Regeneration/Reinigung des Lösungsmittels aufbereitet wird, um eine neue Extraktion des Ausgangsgemischs durchzuführen, und
(g) die wässrige Phase, die das restliche Lactid, das meso-Lactid und die sonstigen Unreinheiten enthält, eventuell nach Reinigung, vor der Lactidsynthese recycelt wird.

9. Verfahren nach Anspruch 1 bis 7, **dadurch gekennzeichnet, dass**:
(i) die flüssige Phase, die sich aus mit Unreinheiten beschicktem Ether zusammensetzt, von Schritt d) auf unter 30 °C bis zum Ausfällen des restlichen Lactitids und meso-Lactids abgekühlt wird, und
(ii)das restliche Lactid und meso-Lactid derart gefiltert werden, dass ein neuer Kuchen und eine flüssige Phase geerntet werden,
(e) die flüssige Phase, die den Ether von Schritt (ii) umfasst, nach Aufbereitung des ladtidreichen Kuchens, in eine Extraktionssäule oder eine Mixer-Settler-Strecke geschickt wird, wo sie mit einer wässrigen Phase in Kontakt versetzt wird, die daraus das meso-Lactid und die sonstigen Unreinheiten aufbereitet,
(f) die Ether-Phase nach oder ohne Absolvieren eines Schritts der Regeneration/Reinigung des Lösungsmittels aufbereitet wird, um eine neue Extraktion des Ausgangsgemischs durchzuführen, und
(g) die wässrige Phase, die das restliche Lactid, das meso-Lactid und die sonstigen Unreinheiten enthält, recycelt wird, eventuell nach Reinigung, vor der Lactidsynthese.

10. Verfahren nach den Ansprüchen 1 bis 7, **dadurch gekennzeichnet, dass**:
(e) die flüssige Phase, die aus mit meso-Lactid und sonstigen Unreinheiten beschicktem Ether zusammengesetzt ist, von Schritt d) nach Rückgewinnung des lactidreichen Kuchens in eine Destillationssäule mit Füllung geschickt wird, wo der Ether am Säulenkopf destilliert wird, wogegen die mit meso-Lactid und sonstigen Unreinheiten beschickte Phase unten aus der Säule austritt,
(f') die mit meso-Lactid und sonstigen Unreinheiten beschickte Phase von Schritt (e') mit Wasser hydrolysiert wird,
(g') der Ether von Schritt (e') aufbereitet wird.

11. Verfahren nach den Ansprüchen 1 bis 7, **dadurch gekennzeichnet, dass**:
(i) die flüssige Phase, die sich aus mit Unreinheiten beschicktem Ether zusammensetzt, von Schritt d) auf unter 30 °C bis zum Ausfällen des restlichen Lactitids und meso-Lactids abgekühlt wird, und
(ii) das restliche Lactid und meso-Lactid derart gefiltert werden, dass ein neuer Kuchen und eine neue flüssige Phase geerntet werden,
(e') die flüssige Phase, die den Ether umfasst, von Schritt ii) nach Aufbereitung des lactidreichen Kuchens in eine Destillationssäule mit Füllung geschickt wird, wo der Ether am Säulenkopf destilliert wird, wogegen die mit meso-Lactid und sonstigen Unreinheiten beschickte Phase unten aus der Säule austritt,
(f) die mit meso-Lactid und sonstigen Unreinheiten beschickte Phase von Schritt (e') mit Wasser hydrolysiert wird,
(g') der Ether von Schritt (e') aufbereitet wird.

12. Verfahren nach einem der vorangehenden Ansprüche, **dadurch gekennzeichnet, dass** der Ether die allgemeine Formel
R¹-O-R²
hat, wobei R¹ und R² unabhängig voneinander eine lineare oder verzweigte Alkyl-oder Alkenylgruppe mit 1 bis 4 Kohlenstoffatomen darstellen,
eine Alcanolgruppe mit 1 bis 4 Kohlenstoffatomen,
eine Arylgruppe,
eine Arylalkylgruppe,
eine Cycloallcylgruppe mit 4 bis 6 Kohlenstoffatomen,
oder bei der R¹ und R² eine zyklische Struktur mit 2 bis 6 Kohlenstoffatomen bilden.
